# EUROPÄISCHE PATENTANMELDUNG

(11) **EP 3 847 986 A1**
(43) Veröffentlichungstag der Anmeldung: **14.07.2021**
(21) Anmeldenummer: 20151509.5
(22) Anmeldetag: 13.01.2020
(51) Int. Cl.: A61B 18/16, A61N 1/04, A61N 1/14, H01B 1/22

(54) **NEUTRALELEKTRODE UND VERFAHREN ZUR AUSBILDUNG EINER SOLCHEN**

(71) Anmelder: Erbe Elektromedizin GmbH, 72072 Tübingen (DE)
(72) Erfinder: Wulff, Erik, 72810 Gomaringen (DE); Felstead, Marcus, 72070 Tuebingen (DE)
(74) Vertreter: Rüger Abel Patentanwälte PartGmbB

(57) **Zusammenfassung**

Eine erfindungsgemäße Neutralelektrode (12) wird an einem Patienten (11) erzeugt, indem eine elektrisch leitfähige und verfestigbare Masse auf die Haut des Patienten aufgetragen wird, die elektrisch leitfähig ist. Dabei kann vor, während oder nach dem Auftragen dieser Masse an dem sich bildenden Elektrodenkörper oder in demselben eingebettet ein elektrisch leitfähiges Inlay (17) angeordnet werden, das mit einem Neutralelektroden-Anschlusskabel (13) verbunden oder verbindbar ist. Das Inlay (17) dient der elektrischen großflächigen Kontaktierung des Elektrodenkörpers (16), der seinerseits einen zuverlässigen elektrischen Kontakt zu dem Patienten (11) herstellt.

## Beschreibung

Die Erfindung betrifft eine Neutralelektrode zur Anbringung an einem Patienten zur Ableitung von elektrochirurgischen Strömen, ein Verfahren zur Ausbildung einer solchen Elektrode sowie ein Produkt zur Verwendung bei der Ausbildung einer solchen Neutralelektrode.

Aus der WO 2004/069070 A1 ist eine Neutralelektrode für die HF-Chirurgie bekannt, die nach Art eines elektrisch ganzflächig oder in Teilflächen leitfähigen Strumpfes ausgebildet ist.

Weiter ist aus der Gebrauchsmusterschrift DE 9101366 U1 eine wiederverwendbare Neutralelektrode bekannt, die einen flächenhaften Träger aus elastischem Silikonkautschuk aufweist. Dieser trägt zwei elektrisch voneinander getrennte, elektrisch leitfähige Elektrodenflächen, die über Anschlussleitungen mit einem Kabel verbunden sind. Über dieses Kabel werden die Elektrodenflächen mit dem Neutralanschluss eines elektrochirurgischen Geräts verbunden,
Ein Überwachungssystem prüft dabei die korrekte Anlage der Elektrodenflächen an dem Patientenkörper.

Eine ähnliche Neutralelektrode ist aus der DE 42 32 255 C1 bekannt. Auch diese weist einen flächenhaften Träger mit mehreren leitfähigen Flächen auf, die Teilelektroden der Neutralelektrode sind. Zur Anbringung am Patienten dient eine Trägerfolie, die beidseitig elektrisch leitfähige Klebefilme aufweist, die eine galvanische Verbindung zwischen den Teilelektroden und dem Patienten herzustellen.

Neutralelektroden müssen einerseits ausreichend groß bemessen sein, um die Stromdichte und damit die Wärmeentwicklung am Patienten unterhalb physiologischer Grenzen zu halten. Andererseits stellt die Anbringung großer Neutralelektroden, insbesondere an Körperstellen größerer Krümmung ein Problem dar. Die Neutralelektrode muss jedenfalls sicher und vollflächig an dem Patienten angebracht sein, um Gewebeschädigungen zu vermeiden. Zur Überwachung der korrekten Anlage weisen manche Neutralelektroden getrennte Teilelektroden auf, die an eine Überwachungseinrichtung angeschlossen sind, die den Betrieb eines elektrochirurgischen Geräts sperrt sobald zwischen beiden Teilelektroden ein Widerstandsgrenzwert überschritten wird.

Aus den obigen Aspekten heraus ergeben sich Restriktionen hinsichtlich der Platzierung der Neutralelektrode am Patienten, so dass manchmal unerwünscht große Abstände zwischen der Neutralelektrode und dem Operationsgebiet vorhanden sind. Außerdem kann die Position des an die Elektrode angeschlossenen Kabels hinderlich sein.

Es ist Aufgabe der Erfindung, ein Konzept für eine Neutralelektrode anzugeben, mit dem ein sicherer elektrischer Kontakt zwischen einer Neutralleitung und dem Patienten geschaffen werden kann und die in Handhabung und Anbringung flexibel ist.

Diese Aufgabe wird mit der Neutralelektrode nach Anspruch **1,** mit dem Verfahren nach Anspruch **12** sowie auch mit dem Produkt nach Anspruch **15** gelöst:

Die erfindungsgemäße Neutralelektrode weist einen Elektrodenkörper aus einem auf die Haut des Patienten in flüssigem, breiigem oder pastösem Zustand aufgetragenen und dort wenigstens teilweise verfestigten, elektrisch leitfähigen Material auf. Das formlose Material wird in nicht verfestigtem Zustand auf den Körper des Patienten aufgebracht und steht deswegen auch ungeachtet etwaiger Körperhaare in innigem Kontakt mit der Haut des Patienten. Auch passt sich die Form des auf den Patienten aufgetragenen Materials jeder Körperform an, sodass jedenfalls eine vollflächige Anlage zu erwarten ist. Außerdem kann durch Applikation einer bedarfsgerechten Menge des formlosen Materials die Elektrodendicke an den jeweiligen Anwendungsfall angepasst werden.

Das aufgetragene Material ist elektrisch leitfähig und dient der Überleitung des elektrochirurgischen Stroms von dem Patienten auf ein Neutralelektroden-Anschlusskabel. Das Material ist, nachdem es unverfestigt aufgetragen worden ist, nach einer Zeit ganz oder teilweise verfestigt, wobei die Verfestigung vorzugsweise mindestens an der vom Patienten abgewandten Seite des Elektrodenkörpers gegeben ist. Beispielsweise kann der Elektrodenkörper aus einem Material bestehen, das oberflächlich abtrocknet und somit an der vom Patienten abgewandten Seite eine Haut oder eine Schutzschicht bildet. Das Material kann sich auch durchgehend verfestigen und auf diese Weise einen vorzugsweise plastisch oder elastisch bleibenden oder auch erstarrten Körper bilden.

Die Verfestigung kann auf Verdunstung von Lösungsmitteln und/oder auf chemischer Reaktion, beispielsweise einer Vernetzungsreaktion beruhen, indem Bestandteile des Materials mit Sauerstoff oder anderen Bestandteilen der Luft reagieren. Auch Feuchtigkeit, beispielsweise von der Haut des Patienten ausgehende Feuchtigkeit und/oder Luftfeuchtigkeit kann einen chemischen Reaktionsprozess in Gang setzen, im Rahmen derer das Material der Elektrode sich beispielsweise vernetzt oder auf anderem Wege verfestigt.

Der Elektrodenkörper enthält ein Inlay, das einen Kabelanschluss aufweist, von dem Elektrodenkörper umschlossen ist oder wenigstens in inniger Berührung mit diesem steht. Dieses Inlay ist vorzugsweise ein elektrisch leitfähiges flächenhaftes Element aus einem Material, dessen spezifische Leitfähigkeit höher ist als die des Materials des Elektrodenkörpers. Das Inlay ist beispielsweise ein Netz, ein Gestrick, ein Gewebe, ein Gitter, eine gelochte Folie, eine ungelochte Folie, eine gelochte oder ungelochte Platte oder dergleichen. Das Inlay wird z.B. bei der Ausbildung der Neutralelektrode am Körper des Patienten angeordnet und dann von dem noch nicht verfestigten Material umhüllt, wonach das aufgetragene Material verfestigt. Somit ist die Neutralelektrode einsatzbereit. Das Inlay hat vorzugsweise einen vorbestimmten Umriss und, auch bei verschiedenen Anwendungen eine immer gleiche Größe. Die Größe der Neutralelektrode kann hingegen variiert werden, indem das formlose Material in größerer oder kleinerer Fläche aufgetragen wird.

Der Elektrodenkörper kann aus einem elektrisch leitfähigen nichtmetallischen Material ausgebildet sein, z.B. aus einem Stoffgemisch, das, zumindest solange der Elektrodenkörper nicht vollständig verfestigt ist, Lösungsmittel enthält. Insbesondere kann das Material als Lösungsmittel, Wasser, Ethanol, Diethylether, Isooctan, Ethylacetat und/oder andere flüchtige, vorzugsweise unpolare flüssige Verbindungen enthalten. Weiter kann das Material ein Polymer enthalten, beispielsweise Polyvinylpyrrolidon, Nitrozellulose, Ethylzellulose und/oder Poly(methylacrylat-isobuten-monoisopropylmaleat), Cyanoacrylat, zum Beispiel 2-Optylcyanoacrylat, Siloxanpolymere, zum Beispiel Hexamethyldisiloxan. Zusätzlich kann das Material zur Herbeiführung oder Verbesserung der elektrischen Leitfähigkeit wässrig gelösten Natriumchlorid oder andere Salze in wässriger Lösung und/oder elektronenleitfähige Partikel, wie beispielsweise Kohlenstoffpartikel, Metallpartikel oder Partikel leitfähiger Keramik enthalten. Der Elektrodenkörper kann somit aus einem Gel mit einer Verdunstungskomponente oder einem Elektrodenkörper mit einer härtenden Komponente sowie einer elektrisch leitfähigen Komponente bestehen. Die Verdunstungskomponenten können leicht flüchtige Lösungsmittel wie z.B. Ethylalkohol, Äther oder ein anderes der oben genannten anderen Lösungsmittel sein. Die härtenden Komponenten können ein oder mehrere der oben genannten vernetzungsfähigen Stoffe sein. Es ist auch möglich, das Material des Elektrodenkörpers in zwei getrennten Komponenten bereit zu stellen, die, wenn sie miteinander in Kontakt gebracht werden, eine sich verfestigende und/oder aushärtende Mischung bilden. Weiter kann das Material zur Ausbildung der Elektrode eine gelbildende Komponente enthalten. Die gelbildende Komponente unterstützt die Ausbildung des Elektrodenkörpers in ausreichender Dicke von vorzugsweise mehr als einem, vorzugsweise mehreren Millimetern. Die gelbildende Komponente ist insbesondere zur Hydrogelbildung eingerichtet, wie z.B. Stärke, Gelatine, Polyacrylsäure oder andere zur Bildung von Carbomeren geeignete Stoffe.

Das Inlay ist vorzugsweise eine Metallstruktur in Gestalt von Folien, Drähten, Netzen oder eines flächenhaften mehrfach durchbrochenen Körpers, wie zum Beispiel eine mehrfach gelochte Metallfolie. Das Inlay kann auch ein metallisierter Kunststoff, metallisiertes Papier oder ein anderer mit einer leitfähigen Beschichtung versehener Körper oder eine einseitig mit Isolierung versehene Metallfolie sein. Insbesondere ist es möglich, das Inlay patientenseitig elektrisch isolierend auszubilden, um einen direkten lokalen Kontakt zwischen hochleitfähigem Metall des Inlays und dem Patienten zu vermeiden. Jedoch steht das Inlay elektrisch mit dem gegebenenfalls etwas weniger leitfähigem Material des Elektrodenkörpers in galvanischem Kontakt, so dass von dem Inlay aufzunehmender Strom von dem Patienten über den Elektrodenkörper übertragen wird.

Ist das Inlay einseitig isoliert, ist es möglich, dass die von dem elektrisch leitfähigen Bereich auf dem nicht metallischen, nicht elektrisch leitfähigen Träger eingenommene Fläche kleiner ist als die des Trägers. Damit überragt der elektrisch isolierende Träger die leitfähigen Bereiche und hält sie von der Haut des Patienten fern. Diese Maßnahme vermeidet lokale Stromkonzentrationen der Haut des Patienten entlang der Kanten der elektrisch leitfähigen Bereiche.

Der Kabelanschluss kann zum lösbaren Verbinden mit einem Kabel eingerichtet oder mit einem Kabelset unlösbar verbunden sein. In letzterem Fall bildet das Kabel einen Teil einer am Patienten zu erzeugende und dann nach Gebrauch insgesamt zu entsorgenden Neutralelektrode.

Das erfindungsgemäße Verfahren zur Anbringung einer Neutralelektrode an einem Patienten umfasst das Anordnen des Inlays an der Haut des Patienten und das Auftragen von formlosem Material, das nach dem Auftragen, wenigstens teilweise, verfestigt oder verfestigt wird. Das Inlay ist dann in den Elektrodenkörper eingebettet. Der Elektrodenkörper ist nach dem teilweisen Verfestigen außen nicht mehr klebrig und zumindest im Randbereich mit der Haut des Patienten verklebt. Innen kann er auch verfestigt oder auch von gelartiger Konsistenz sein. Dies fördert die Flexibilität, den elektrischen Kontakt und erleichtert die spätere Entfernung der Neutralelektrode von der Haut des Patienten.

Vor dem Schritt des Anbringens des Inlays an dem Patienten kann bereits das formlose Material oder ein Teil desselben auf die Haut desselben aufgebracht werden, so dass das Inlay an der Hautoberfläche klebt. Ist die aufgetragene Schicht aus formlosem verfestigbaren Material ausreichend dick, um von einem Inlay ausgehenden Strom zu verteilen, kann das Inlay an dem Material, wie an einem dick aufgetragenen Klebstoff angebracht werden. Es kann auf den Auftrag weiteren Materials verzichtet werden. Es bildet sich dann eine Neutralelektrode, deren Metallinlay an der Außenseite der Elektrode angeordnet ist. Der Vorteil dieses Verfahrens liegt in der Abdeckung des noch nicht verfestigten Elektrodenkörpers nach außen.

Die erfindungsgemäße Neutralelektrode wird vorzugsweise unter Nutzung eines Produkts erzeugt, das zur Ausbildung der Neutralelektrode an einem Patienten eigens bereit gestellt ist. Das Produkt umfasst eine formlose in einem Gefäß gehaltene Masse, die elektrisch leitfähig und verfestigbar ist. Diese Masse bildet nach Auftrag auf die Haut des Patienten das Material, aus dem der Elektrodenkörper besteht. Die Masse ist vorzugsweise flüssig, breiig oder pastös und elektrisch extrinsisch oder intrinsisch leitfähig. Beispielsweise kann sie elektrisch leitfähige Partikel und/oder dissoziierte Salze, zum Beispiel wässrig gelöstes NaCl enthalten. Zu dem Gefäß gehört vorzugsweise eine Auftrageeinrichtung, beispielsweise in Gestalt eines Sprühkopfs, eines Spenders mit Pumpe, ähnlich einem Seifenspender, eines mit einem Pinsel versehenen Deckels oder eines dem Gefäß beigegebenen Spatels, Pinsels, Rollers oder dergleichen. Außerdem kann das Produkt zu einem Set gehören, zu dem neben dem erwähnten Gefäß, oder mehrere Inlays und gegebenenfalls ein oder mehrere Neutralelektroden-Anschlusskabel gehören.

Unterschiedliche Größen von Neutralelektroden können durch unterschiedlich verwendete Mengen der z.B. gelförmigen formlosen Masse realisiert. Die verwendete Menge richtet sich nach der geplanten Anwendung bzw. Leistung des elektrochirurgischen Generators. Der Generator kann dabei darauf eingerichtet sein, in Abhängigkeit der Benutzereinstellungen (Mode, Effektstufe, etc.) Vorgaben für die Menge des Gels bzw. notwendige Größe der Neutralelektrode zu machen.

Weitere Einzelheiten vorteilhafter Ausführungsformen der Erfindung ergeben sich aus Unteransprüchen, aus der Zeichnung oder aus der Beschreibung. Es zeigen:
Figur 1 einen Patienten mit an ihm angebrachter Neutralelektrode, in schematisierter Darstellung,
Figur 2 den Patienten mit angebrachter Neutralelektrode, in ausschnittsweiser Schnittdarstellung,
Figur 3 ein zu der Neutralelektrode nach Figur 2 gehöriges Inlay, in schematischer Draufsicht,
Figur 4 die Neutralelektrode nach Figur 2, in vergrößerter schematisierter Schnittdarstellung,
Figur 5 eine weiter vergrößerte ausschnittsweise Schnittdarstellung der Neutralelektrode nach Figur 4,
Figur 6 eine noch weiter vergrößerte ausschnittsweise Darstellung eines Teils des Elektrodenkörpers mit elektrisch leitfähigen Partikeln,
Figur 7 ein Gefäß mit darin gehaltener Masse zur Ausbildung der Neutralelektrode als Sprühflasche, in Vertikalschnittdarstellung,
Figur 8 ein Gefäß mit darin befindlicher Masse und Deckelpinsel, in Vertikalschnittdarstellung,
Figur 9 eine abgewandelte Ausführungsform einer erfindungsgemäßen Neutralelektrode, in Längsschnittdarstellung,
Figur 10 eine weiter abgewandelte Ausführungsform einer Neutralelektrode, in schematisierter Längsschnittdarstellung.

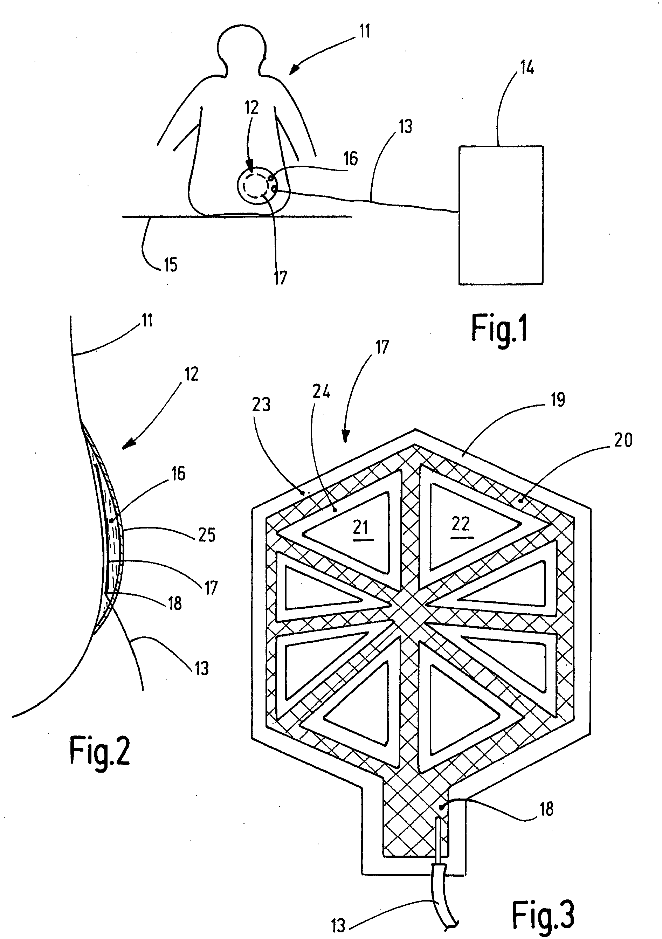

In Figur 1 ist eine an einem Pateinten 11 angebrachte Neutralelektrode 12 veranschaulicht, die über ein Neutralelektroden-Anschlusskabel 13 mit einem beispielsweise der Elektrochirurgie dienenden Gerät 14 verbunden ist. Die Anbringung der Neutralelektrode 12 an dem Patienten 11 kann beispielsweise bei der Vorbereitung des Patienten 11 zur Operation erfolgen, während dieser auf einer Unterlage 15 sitzt oder liegt. Die Platzierung der Neutralelektrode 12 kann sich dabei nach medizinischen oder operationstechnischen Gesichtspunkten richten. Auf Körperrundungen, Falten oder dergleichen, muss keine gesonderte Rücksicht genommen werden.

Figur 2 veranschaulicht die Neutralelektrode 12 an einer gekrümmten Hautpartie des Patienten 11. Die Neutralelektrode 12 besteht aus einem Elektrodenkörper 16 und einem darin eingebetteten Inlay 17. Letzteres ist an einem Kabelanschluss 18, der auch aus Figur 3 ersichtlich ist, mit dem Neutralelektroden-Anschlusskabel 13 elektrisch verbunden. Der Kabelanschluss 18 kann durch eine feste elektrisch leitfähige Verbindung des Leiters des Neutralelektroden-Anschlusskabels 13 mit dem Inlay 17 oder durch eine lösbare Verbindung, wie beispielsweise einen Druckknopf, eine Klemme oder dergleichen gebildet sein.

Das Inlay 17 ist ein wenigstens teilweise elektrisch leitfähiges Element, das vorzugsweise wenigstens eine Fläche umschreibt, die so groß ist, dass der durch das Neutralelektroden-Anschlusskabel 13 abzuleitende Strom geteilt durch die von dem Inlay umschriebene Fläche einen physiologischen Stromdichtegrenzwert unterschreitet. Im einfachsten Fall kann das Inlay 17 ein beispielweise zu einem Ring oder einer anderen geeigneten Form gebogener abisolierter Abschnitt des Leiters des Neutralelektroden-Anschlusskabels 13 sein. Es kann aber auch ein gesondertes Element aus Metall oder einem anderen elektrisch leitfähigen Material wie z.B. leitfähigem Gummi sein, das eine Fläche überspannt. Diese Fläche kann z.B. etwa handtellergroß sein.

Im vorliegenden Ausführungsbeispiel ist das Inlay 17 im Umriss etwa sechseckig und besteht aus einem elektrisch nicht leitenden Träger 18, beispielsweise aus Kunststofffolie, Papier oder dergleichen. Auf diesem Träger ist vorzugsweise an der patientenabgewandten Seite ein elektrischer Leiter 20 angebracht, der mit dem Kabelanschluss 18 galvanisch verbunden ist. Vorzugsweise werden der Kabelanschluss 18 und der Leiter 20 durch eine dünne zusammenhängend ausgebildete Metallschicht ausgebildet, die zum Beispiel aus streifenförmigen Abschnitten besteht, die zusammen ein Netz bilden. Die streifenförmigen Abschnitte erstrecken sich vorzugsweise um wenigstens eine Öffnung 21, sowie gegebenenfalls mehrere Öffnungen herum, die in dem Träger 19 ausgebildet sind. Die streifenförmigen Leiterbereiche sind dabei vorzugsweise schmaler als die entsprechenden Bereiche des Trägers 19, so dass außen um den Leiter herum sowie um jede Öffnung 21, 22 herum Abstandsbereiche 23, 24 definiert sind.

An der dem Patienten 11 zugewandten Seite kann der Träger 19 mit einer elektrisch nicht leitfähigen oder auch einer elektrisch leitfähigen Haftstoffschicht versehen sein. Der spezifische Leitwert dieser Haftstoffschicht ist vorzugsweise geringer als die spezifische Leitfähigkeit von Metall, weiter vorzugsweise aber größer als die spezifische elektrische Leitfähigkeit des Elektrodenkörpers. Diese Maßnahme sorgt für eine gleichmäßige Stromdichteverteilung unter der Neutralelektrode 12.

Der an dem Patienten 11 zur Ausbildung der Neutralelektrode 12 ausgebildete Elektrodenkörper 16 überdeckt das Inlay 17 und klebt an der Haut des Patienten 11. Der Elektrodenkörper 12 berührt dabei die Haut des Patienten 11 in einem das Inlay 17 umschließenden Bereich. Außerdem durchsetzt der Elektrodenkörper 16 die Öffnungen 21, 22 und berührt auch dort die Haut des Patienten 11.

Der Elektrodenkörper 16 besteht vorzugsweise aus einem elektrisch leitfähigen an der Luft verfestigbaren Material, das zumindest an der der Luft zugewandten, vom Patienten 11 abgewandten Seite eine verfestigte, nicht klebrige Schicht oder Haut 25 bildet. Unterhalb dieser Schicht 25 kann das Material des Elektrodenkörpers 16 ebenfalls verfestigt oder auch weniger oder nicht verfestigt sein. Figur veranschaulicht diesen Aufbau schematisch zu Beginn der Verfestigung des Elektrodenkörpers 16. Die Schicht 25 haftet an ihrem Rand an der Haut des Patienten. Sie kann eine durch Trocknung, d.h. Verdunstung eines Lösungsmittels oder durch Vernetzung eines Polymers verfestigte Schicht sein. Beispielsweise kann der Elektrodenkörper 16 unter anderem eine alkoholischen Acrylat-Kopolymer/Polyurethan-Lösung enthalten, die zusätzlich Dimethyläther enthalten kann. Durch Verflüchtigung des enthaltenen Ethanols und/oder des Dimethyläthers verfestigt sich der Elektrodenkörper 16 von außen nach innen, wobei die verfestigte Zone bis zu dem Inlay 17 und gegebenenfalls auch bis zu der Haut des Patienten 11 gehen kann. Es können aber auch unverfestigte Bereiche bestehen bleiben, was das spätere Ablösen der Neutralelektrode nach Gebrauch erleichtert.

Das Inlay 17 kann, wie erwähnt, mittels einer Klebeschicht 26 an der Haut des Patienten befestigt worden sein. Es ist aber auch möglich, von einer gesonderten Befestigung des Inlays 17 an der Haut des Patienten 11 abzusehen. Dies ist insbesondere angeraten, wenn das Inlay aus lediglich schmalen Streifen oder Drähten besteht, z.B. in Form eines Gewebes, Netzes oder Gestricks.

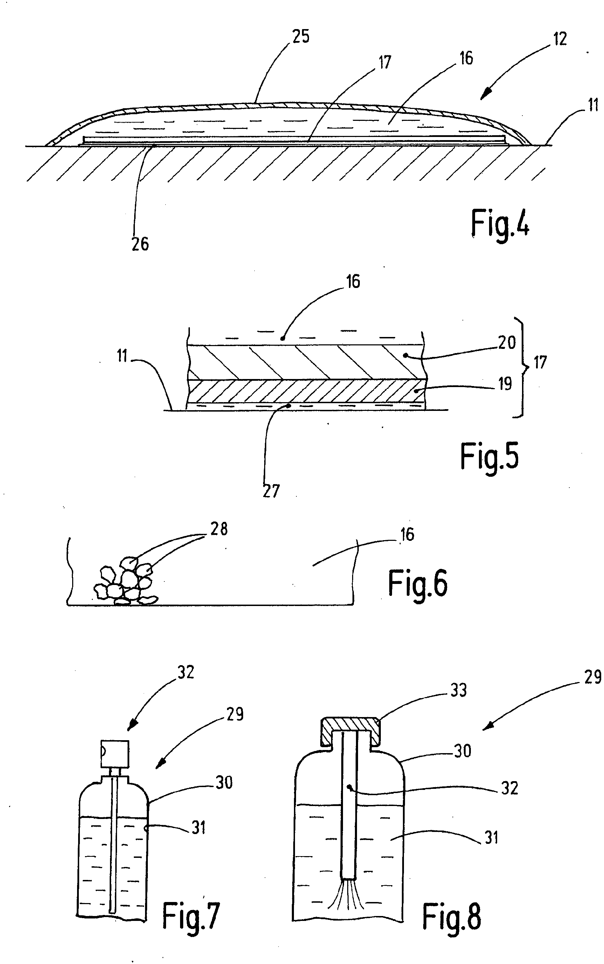

Es ist auch möglich, das Material des Elektrodenkörpers 16 selbst zur Befestigung des Inlays 17 zu nutzen. Dazu veranschaulicht Figur 5 einen Ausschnitt aus der Neutralelektrode 12 in vergrößerter Schnittdarstellung. Der Elektrodenkörper 16 durchsetzt hier das Inlay 17 und bildet unterhalb desselben zwischen dem Inlay 17 und dem Patienten 11 eine dünne schichtartige Zone 27, die elektrisch leitfähig ist und vollflächig an dem Patienten 11 haftet. Diese Ausführungsform bietet eine verbesserte Stromverteilung gegenüber der Ausführungsform nach Figur 4, wenn letztere einen elektrisch nicht leitenden Klebstoff 26 nutzt oder mit einem Inlay 17 ohne direkte Befestigung an der Haut des Patienten 11 arbeitet.

Das Material des Elektrodenkörpers 16 kann intrinsisch leitfähig sein, indem es beispielsweise wässrig gelöstes Natriumchlorid, Essigsäure oder andere ionenleitfähige Stoffe, wie wässrige Seifenlösung oder dergleichen enthält. Das Material des Elektrodenkörpers 16 kann jedoch auch extrinsisch leitfähig sein, wie es Figur 6 in sehr stark vergrößerter Form veranschaulicht. Das Material des Elektrodenkörpers 16 bildet eine Matrix, die mit elektrisch leitfähigen Partikeln 28, beispielsweise Metallpartikeln, Partikeln leitfähiger Keramik, Ruß, Graphitpulver oder dergleichen gefüllt ist. Das Material des Elektrodenkörpers kann auch durch eine Kombination vorgenannter Zusammensetzungen gekennzeichnet sein. Z.B. kann eine Mischung aus ionenleitfähigem Material und leitfähigen Partikeln vorgesehen sein. Das Material kann somit sowohl extrinsisch als auch intrinsisch leitfähig sein. Damit kann eine hohe spezifische Leitfähigkeit erreicht werden, die insbesondere höher ist als die spezifische Leitfähigkeit der Haut. Dadurch bildet die Neutralelektrode 12 eine Äquipotentialfläche.

Figur 7 veranschaulicht eine Packung 29, die mit dem Inlay 17 nach Figur 3 ein Set bilden kann. Die Packung 29 umfasst ein als Sprühflascheoder Spender ausgebildetes Gefäß 30, das eine formlose vorzugsweise flüssige Masse 31 beherbergt. Die Masse 1 ist beispielsweise eine alkoholische Acrylat-Copolymer/Polyurethan-Lösung. Das nach außen geschlossene Gefäß 30 ist mit einer Auftrageeinrichtung 32, beispielsweise in Gestalt eines Sprühkopfs versehen. Ist das Gefäß 30 mit Druckgas oder einem sonstigen Treibmittel gefüllt, enthält die Auftrageeinrichtung 32 ein Ventil und eine Zerstäubervorrichtung. Ist das Gefäß 30 hingegen drucklos, umfasst die Auftrageeinrichtung 32 eine Pumpvorrichtung und eine Dispensereinrichtung. Diese kann als einfache Runddüse, Schlitzdüse, als Brausekopf oder als Zerstäubereinrichtung ausgebildet sein.

Die formlose Masse 31 kann auch eher breiiger Konsistenz sein. Dazu veranschaulicht Figur 8 ein Gefäß 30 mit einem Verschlussdeckel 33, an dem ein als Auftrageeinrichtung 32 dienender Pinsel befestigt ist. Die hier enthalten Masse 31 kann zusätzlich zu den genannten Bestandteilen konsistenzgebende Bestandteile, insbesondere quellfähige Bestandteile, wie Stärke, Gelbildner oder dergleichen, enthalten, um einen möglichst dickschichtigen Auftrag der formlosen Masse auf die Haut des Patienten zu ermöglichen, so dass der sich ausbildende Elektrodenkörper nicht lediglich als dünner Film, sondern als schwartenartige Haut vorliegt.

Das Ausbilden der Neutralelektrode an dem Patienten 11 kann wie folgt geschehen:
Ist das Inlay 17 an wenigstens einer Seite mit einem elektrisch leitfähigen oder auch einem elektrisch isolierenden Klebstoff versehen, kann das Inlay 17 zunächst an der Haut des Patienten 11 angebracht werden, wonach die Masse 31 aus der Packung 29 nach Figur 7 oder 8 auf das Inlay 17 und dabei dieses überdeckend aufgebracht wird. Nach zumindest oberflächlicher Verfestigung der aufgebrachten Masse ist die Neutralelektrode 19 einsatzbereit.

In einer zweiten Variante, die im Zusammenhang mit Figur 5 bereits angedeutet ist und die sich insbesondere eignet, wenn das Inlay 17 patientenseitig keine Klebstoffbeschichtung aufweist, wird zunächst eine dünne Schicht der formlosen Masse auf die Haut des Patienten aufgetragen. Danach wird das Inlay 17 aufgesetzt (Figur 5) und im Weiteren formlosen Masse aufgetragen, bis der Elektrodenkörper 16 in der gewünschten Schichtdicke vorhanden ist.

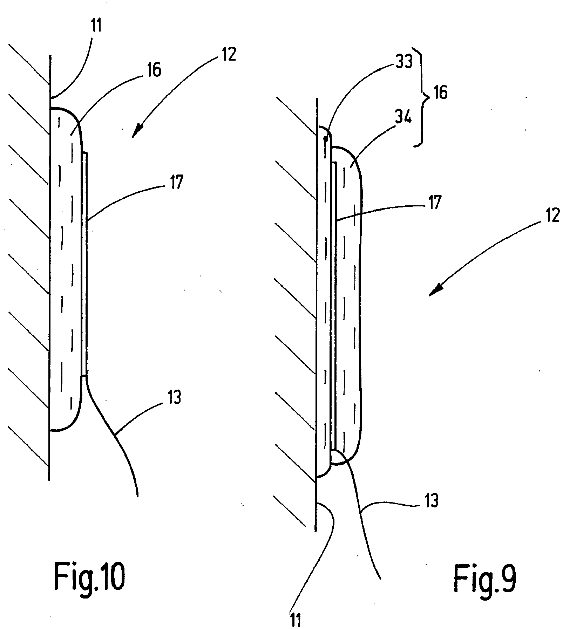

Für Inlays 17 ohne patientenseitige Isolierung, eignen sich insbesondere die Varianten nach Figur 9 und 10. Dazu wird beispielsweise gemäß Figur 9 zunächst eine erste Lage 33 des Elektrodenkörpers 16 auf der Haut des Patienten 11 angeordnet und vor Abtrockung oder anderweitiger Verfestigung derselben das Inlay 17 angebracht. Dieses wird dann mit einer zweiten Lage 34 des Materials des Elektrodenkörpers 16 überdeckt, so dass dieser letztendlich das Inlay 17 umschließend ausgebildet ist.

Es ist im Weiteren auch möglich, den Elektrodenkörper 16 gemäß Figur 10 in einem ersten Schritt auf die Haut des Patienten 11 aufzutragen und das Inlay 17 dann äußerlich an dem noch haftfähigen Elektrodenkörper 16 anzubringen. Es bildet sich eine Neutralelektrode mit außen liegendem Inlay.

Allen vorgenannten Ausführungsformen und Varianten ist gemeinsam, dass die Neutralelektrode 12 zusätzlich mit einem Temperatursensor oder anderen Sensoren zur Überwachung der Stromdichte, der Temperatur oder andere physikalischer Größen versehen werden kann. Solche Sensoren können an den Inlays 17 angebracht oder gesondert in den Elektrodenkörper 16 eingebettet sein.

Es können Sets bereitgestellt werden, die eine oder mehrere Packungen 29 und ein oder mehrere Inlays 17 umfassen. Die Anzahl der Inlays 17 kann größer sein als die Anzahl der Packungen 29. Auch können Inlays 17 verschiedener Form oder Größe bereitgestellt werden, um verschiedene Einsatzfälle abzudecken. Zusätzlich kann dem Set eine Schablone beigegeben sein, die beim Aufsprühen auf Pinseln oder anderweitigen Auftragen der Masse 31 die äußere Berandung des Auftragsgebiets begrenzen und somit die Form der Neutralelektrode 12 bestimmen kann. Es können zu dem Set auch ein oder mehrere Sensoren, z.B. Temperatursensoren gehören.

Eine erfindungsgemäße Neutralelektrode 12 wird an einem Patienten 11 erzeugt, indem eine elektrisch leitfähige und verfestigbare Masse auf die Haut des Patienten 11 aufgetragen wird, die elektrisch leitfähig ist. Dabei kann vor, während oder nach dem Auftragen dieser Masse an dem sich bildenden Elektrodenkörper 16 oder in demselben eingebettet ein elektrisch leitfähiges Inlay 17 angeordnet werden, das mit einem Neutralelektroden-Anschlusskabel 13 verbunden oder verbindbar ist. Das Inlay 17 dient der elektrischen großflächigen Kontaktierung des Elektrodenkörpers 16, der seinerseits einen zuverlässigen elektrischen Kontakt zu dem Patienten 11 herstellt.

Wird eine Neutralelektrodenüberwachung gewünscht, können auf diese Weise auch zwei voneinander beabstandete Neutralelektroden an der Haut des Patienten 11 angebracht werden, die jeweils separat mit entsprechenden Neutralelektrodenanschlüssen oder einem kombinierten Anschluss eines überwachenden Geräts 14 verbunden werden. Dieses kann beide Elektroden gleichrangig zur Stromausleitung aus dem Patienten nutzen und durch Widerstandsüberwachung zwischen den Elektroden die korrekte Funktion und Kontaktierung am Patienten prüfen.

### Bezugszeichen:

- 11: Patient
- 12: Neutralelektrode
- 13: Neutralelektroden-Anschlusskabel
- 14: Gerät
- 15: Unterlage
- 16: Elektrodenkörper
- 17: Inlay
- 18: Kabelanschluss
- 19: Träger
- 20: Leiter
- 21, 22: Öffnungen
- 23, 24: Abstandsbereiche
- 25: Schicht
- 26: Klebstoff
- 27: Zone
- 28: Partikel
- 29: Packung
- 30: Gefäß
- 31: Masse
- 32: Auftrageeinrichtung
- 33, 34: Lagen

## Patentansprüche

1. Neutralelektrode (12) zur Anbringung an einem Patienten (11) zur Ableitung von elektrochirurgischen Strömen,
mit einem Elektrodenkörper (16) aus einem auf die Haut des Patienten (11) aufgetragenen und dort wenigstens teilweise verfestigten Material, und
mit einem Inlay (17), das einen Kabelanschluss (18) aufweist und von dem Elektrodenkörper (16) umschlossen ist.

2. Neutralelektrode nach Anspruch 1, **dadurch gekennzeichnet, dass** der Elektrodenkörper (16) aus einem elektrisch leitfähigen nichtmetallischen Material ausgebildet ist.

3. Neutralelektrode nach einem der Ansprüche 1 oder 2, **dadurch gekennzeichnet, dass** der Elektrodenkörper (16) aus einem Gel mit einer Verdunstungskomponente besteht.

4. Neutralelektrode nach einem der vorstehenden Ansprüche, **dadurch gekennzeichnet, dass** der Elektrodenkörper (16) eine härtende Komponente enthält.

5. Neutralelektrode nach einem der vorstehenden Ansprüche, **dadurch gekennzeichnet, dass** der Elektrodenkörper (16) eine lufthärtende Komponente enthält.

6. Neutralelektrode nach einem der vorstehenden Ansprüche, **dadurch gekennzeichnet, dass** der Elektrodenkörper (16) ein ionenleitfähiges Material umfasst.

7. Neutralelektrode nach einem der vorstehenden Ansprüche, **dadurch gekennzeichnet, dass** der Elektrodenkörper (16) elektronenleitfähige Partikel (28) umfasst.

8. Neutralelektrode nach einem der vorstehenden Ansprüche, **dadurch gekennzeichnet, dass** das Inlay (17) eine Metallstruktur ist.

9. Neutralelektrode nach einem der vorstehenden Ansprüche, **dadurch gekennzeichnet, dass** das Inlay (17) patientenseitig isoliert ist.

10. Neutralelektrode nach einem der vorstehenden Ansprüche, **dadurch gekennzeichnet, dass** der Kabelanschluss (18) zum lösbaren Verbinden mit einem Neutralkabel (13) eingerichtet oder mit einem Neutralelektroden-Anschlusskabel (13) fest verbunden ist.

11. Neutralelektrode nach einem der vorstehenden Ansprüche, **dadurch gekennzeichnet, dass** das Inlay (17) mit einem Temperaturfühler oder einem sonstigen Sensor verbunden ist.

12. Verfahren zur Anbringung einer Neutralelektrode an einem Patienten (11) zur Ableitung von elektrochirurgischen Strömen,
wobei bei dem Verfahren ein Elektrodenkörper (16) auf der Haut des Patienten (11) ausgebildet wird, indem auf dessen Haut eine formloses Masse (31) aufgetragen und dort wenigstens teilweise verfestigt wird.

13. Verfahren nach Anspruch 12, **dadurch gekennzeichnet, dass** vor dem Aufbringen des Elektrodenkörpers (16) auf die Haut des Patienten (11) ein Metallinlay aufgelegt wird, auf das die formlose Masse (31) aufgebracht wird.

14. Produkt zur Ausbildung einer Neutralelektrode (12) an einem Patienten (11) zur Ableitung von elektrochirurgischen Strömen, bestehend:
aus einer formlosen, in einem Gefäß (30) gehaltenen Masse (31), die elektrisch leitfähig und verfestigbar ist.

15. Produkt nach Anspruch 14, **dadurch gekennzeichnet, dass** das Gefäß (30) mit einer Auftrageeinrichtung (32) versehen ist.
